# EUROPEAN PATENT APPLICATION

(11) **EP 2 777 768 A1**
(43) Date of publication of application: **17.09.2014**
(21) Application number: 14159611.4
(22) Date of filing: 13.03.2014
(51) Int. Cl.: A61N 5/10

(54) **Method and apparatus pertaining to irradiating a treatment volume while maintaining the treatment volume a predetermined offset distance from the radiation-treatment isocenter**

(30) Priority: 15.03.2013 US 201313835137
(71) Applicant: Varian Medical Systems Technologies, Inc., Palo Alto, CA 94304-1038 (US)
(72) Inventor: Etmektzoglou, Athanasios, Milpitas, CA California 95035 (US)
(74) Representative: Foster, Mark Charles

(57) **Abstract**

A control circuit 109 is configured to control selective movement of at least the patient-support platform 108 (during a radiation treatment session and typically while the radiation source 102 is also moving) such that during at least a part of the radiation treatment session a patient's radiation-treatment target volume is maintained at a predetermined offset distance away from the radiation-treatment isocenter. By one approach the control circuit maintains that predetermined offset distance for the duration of the radiation treatment session. By one approach, the control circuit is further configured to control selective movement of at least the patient-support platform such that during the radiation treatment session the patient's radiation-treatment target volume moves along a predetermined trajectory (even while maintaining the aforementioned predetermined offset distance).

## Description

### Technical Field

This invention relates generally to the therapeutic irradiation of a patient's target volume via a moving source of radiation.

### Background

The use of radiation to treat medical conditions comprises a known area of prior art endeavor. For example, radiation therapy comprises an important component of many treatment plans for reducing or eliminating unwanted tumors. Unfortunately, applied radiation does not inherently discriminate between unwanted materials and adjacent healthy tissues, organs, or the like that are desired or even critical to continued survival of the patient. As a result, radiation is ordinarily applied in a carefully administered manner to at least attempt to restrict the radiation to a given target volume.

Many treatment plans provide for delivering radiation towards a target tissue from a plurality of different angles. Arc therapy, for example, comprises one such approach. Generally speaking, such an approach has the benefit of tending to provide a desired radiation dose to the target volume while lessening the overall radiation impact on any particular non-targeted material by spreading the collateral radiation dose over a larger body of non-targeted material.

That said, such approaches can still potentially result in delivering a particularly sensitive non-targeted area of the patient's body with an undue radiation dose (either as a single dose or as an aggregated dose that accumulates, for example, over a sequence of radiation treatments).

### Summary

The present invention provides a radiation-therapy apparatus, a radiation-therapy treatment-planning apparatus and a method as defined in the claims.

In another aspect, the present invention provides a method comprising:
by a control circuit for a radiation-therapy machine having a movable radiation source and a corresponding radiation-treatment isocenter and during a radiation-treatment session for a patient:
   controlling selective movement of a patient-support platform such that during at least a part of the radiation treatment session a patient's radiation-treatment target volume is maintained at a predetermined offset distance away from the radiation-treatment isocenter.

In this method maintaining the predetermined offset distance may comprise maintaining the predetermined offset distance throughout the radiation treatment session for the patient.

The method may further comprise: controlling selective movement of the patient-support platform such that during the radiation treatment session the patient's radiation-treatment target volume moves along a predetermined trajectory.

In this method the predetermined trajectory may be constrained to a preselected geometric volume.

In this method the preselected geometric volume may have its geometric center disposed coincident with the radiation-treatment isocenter.

### Brief Description of the Drawings

The above needs are at least partially met through provision of the method and apparatus pertaining to irradiating a treatment volume while maintaining the treatment volume a predetermined offset distance from the radiation-treatment isocenter described in the following detailed description, particularly when studied in conjunction with the drawings, wherein:
FIG. 1 comprises a block diagram-schematic view as configured in accordance with various embodiments of the invention;
FIG. 2 comprises a perspective schematic view as configured in accordance with various embodiments of the invention;
FIG. 3 comprises a perspective schematic view as configured in accordance with the prior art;
FIG. 4 comprises a perspective schematic view as configured in accordance with various embodiments of the invention;
FIG. 5 comprises a perspective schematic view as configured in accordance with various embodiments of the invention;
FIG. 6 comprises a perspective schematic view as configured in accordance with various embodiments of the invention;
FIG. 7 comprises a perspective schematic view as configured in accordance with various embodiments of the invention;
FIG. 8 comprises a perspective schematic view as configured in accordance with various embodiments of the invention;
FIG. 9 comprises a perspective schematic view as configured in accordance with various embodiments of the invention;
FIG. 10 comprises a perspective schematic view as configured in accordance with various embodiments of the invention; and
FIG. 11 comprises a block diagram as configured in accordance with various embodiments of the invention.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present invention. Also, common but well-understood elements that are useful or necessary in a commercially feasible embodiment are often not depicted in order to facilitate a less obstructed view of these various embodiments of the present invention. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein.

### Detailed Description

Generally speaking, these various embodiments presume use of a (1) radiation-therapy machine having a movable radiation source and a corresponding radiation-treatment isocenter and (2) a patient-support platform. A control circuit is configured to control selective movement of at least the patient-support platform (during a radiation treatment session and typically while the radiation source is also moving) such that during at least a part of the radiation treatment session a patient's radiation-treatment target volume is maintained at a predetermined offset distance away from the radiation-treatment isocenter.

By one approach the control circuit maintains that predetermined offset distance for the duration of the radiation treatment session. The predetermined offset distance can vary with the application setting. Example offset distances of possible value, however, can range from, by way of example, around 2 centimeters to around 20 centimeters (with longer distances certainly being possible and with a distance of around 10 centimeters being useful for at least some application settings).

By one approach, the control circuit is further configured to control selective movement of at least the patient-support platform such that during the radiation treatment session the patient's radiation-treatment target volume moves along a predetermined trajectory with respect to the radiation-treatment isocenter (even while maintaining the aforementioned predetermined offset distance). It can be useful in at least some application settings to constrain that predetermined trajectory to a preselected geometric volume. These teachings are highly flexible in practice and will accommodate a variety of differently-shaped geometric volumes. For many application settings, and for ease of calculation, it can be useful to use a sphere as the predetermined geometric volume (where the sphere can have its center coincident with the aforementioned radiation-treatment isocenter and its radius equal to the aforementioned predetermined offset distance).

So configured, and contrary to typical practice in these regards, the treatment volume can be exposed to radiation via a plurality of planes instead of via only a single plane during a single radiation-treatment session. Accordingly, these teachings permit a given radiation treatment plan greater flexibility to avoid the undue irradiation of non-targeted areas including particularly critical or susceptible non-targeted areas.

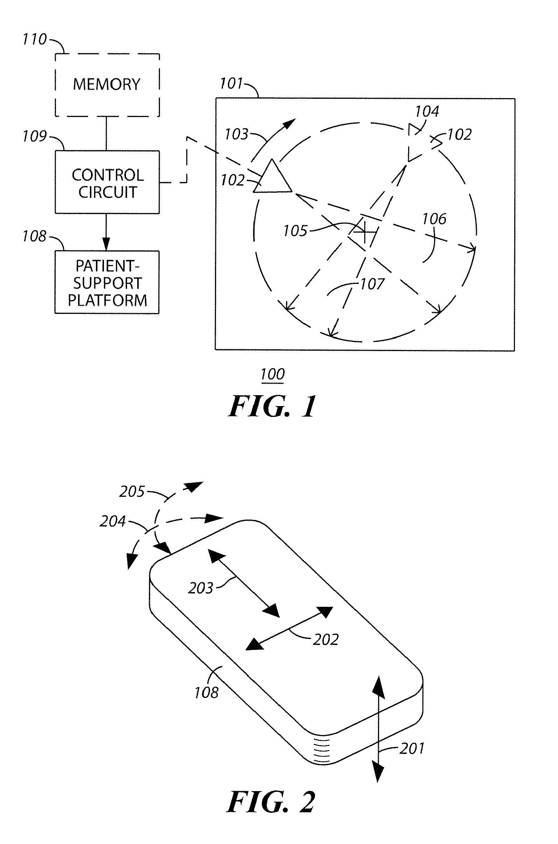

These and other benefits may become clearer upon making a thorough review and study of the following detailed description. Referring now to the drawings, and in particular to FIG. 1, the present teachings pertain to a radiation-therapy apparatus 100 having a radiation-therapy machine 101 having a movable radiation source 102 (such as a moving source of X-rays). In this example the radiation source 102 moves (as suggested by the arrow bearing the reference numeral 103) along an arcuate pathway comprising the circumference of a circular track. In this particular example the radiation source 102 has full freedom of motion throughout the 360 degrees of the circular track. Regardless of its location, however, the radiation source 102 remains aimed at the center of the circle. This center is known in the art as the radiation-treatment isocenter and is denoted in these figures by reference numeral 105.

This description presumes that the radiation source 102 outputs radiation as a so-called fan beam. One such fan beam is denoted by reference numeral 106 and another such fan beam (as output by the radiation source 102 when the latter is positioned in another location denoted by reference numeral 104) is denoted by reference numeral 107. (It will be understood that any of a variety of known beam-shaping tools (such as but not limited to adjustable jaws and multi-leaf collimators) can be used to adjust these fan beams narrower or wider than as drawn as appropriate to the needs of a given treatment plan.)

Generally speaking, this description presumes that the radiation-therapy machine 101 is of sufficient size to receive at least a portion of a patient during the administration of a radiation dose. There are numerous such radiation-therapy machines known in the art. Generally speaking, their construction and manner of use comprises a well-understood area of prior art endeavor. Accordingly, for the sake of brevity and simplicity further description in these regards will not be provided here except where additional details may be particularly relevant to a particular description provided further herein.

The radiation-therapy apparatus 100 further comprises a patient-support platform 108. Referring momentarily to FIG. 2, the patient-support platform 108 can comprise, if desired, a so-called couch. Various couches are known in the art for this very purpose and the present teachings will accommodate essentially any form factor or design that will otherwise serve the purposes set forth herein.

This patient-support platform 108 is sized and configured to fit at least partially within the aforementioned radiation-therapy machine 101. The patient-support platform 108 is also configured to selectively move relative to the radiation-therapy machine 101. The specific available directions of movement can vary to some extent with the application setting. Examples of potentially useful directions of relative movement include, but are not limited to, vertical movement as denoted by reference numeral 201, lateral horizontal movement as denoted by reference numeral 202, and longitudinal horizontal movement as denoted by reference numeral 203. Other examples include yaw movement (i.e., rotational movement about a vertical axis) as denoted by reference numeral 204 and pitch movement as denoted by reference numeral 205.

The present teachings are highly flexible in practice and will readily accommodate other kinds of movement by the patient-support platform 108 when available. For example, an ability of the patient-support platform 108 to roll with respect to the longitudinal axis could serve in these regards. These teachings will also readily accommodate the use of a patient-support platform 108 that comprises two or more separate patient-support components that can each move, at least to some extent, independently of one another.

Referring again to FIG. 1, the radiation-therapy apparatus 100 further includes a control circuit 109 that operably couples to at least the patient-support platform 108. So configured the control circuit 109 is able to control the selective movement of the patient-support platform 108. Such a control circuit 109 can comprise a fixed-purpose hard-wired platform or can comprise a partially or wholly programmable platform. These architectural options are well known and understood in the art and require no further description here. This control circuit 109 is configured (for example, by using corresponding programming as will be well understood by those skilled in the art) to carry out one or more of the steps, actions, and/or functions described herein.

By one optional approach the control circuit 109 also operably couples to a memory 110. The memory 110 may be integral to the control circuit 109 or can be physically discrete (in whole or in part) from the control circuit 109 as desired. This memory 110 can also be local with respect to the control circuit 109 (where, for example, both share a common circuit board, chassis, power supply, and/or housing) or can be partially or wholly remote with respect to the control circuit 109 (where, for example, the memory 110 is physically located in another facility, metropolitan area, or even country as compared to the control circuit 109).

This memory 110 can serve, for example, to non-transitorily store the computer instructions that, when executed by the control circuit 109, cause the control circuit 109 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as an erasable programmable read-only memory (EPROM).)

If desired, this control circuit 109 can also operably couple to the aforementioned radiation-therapy machine 101 to thereby control, for example, the operation and/or movement of the radiation source 102 during a radiation-treatment session.

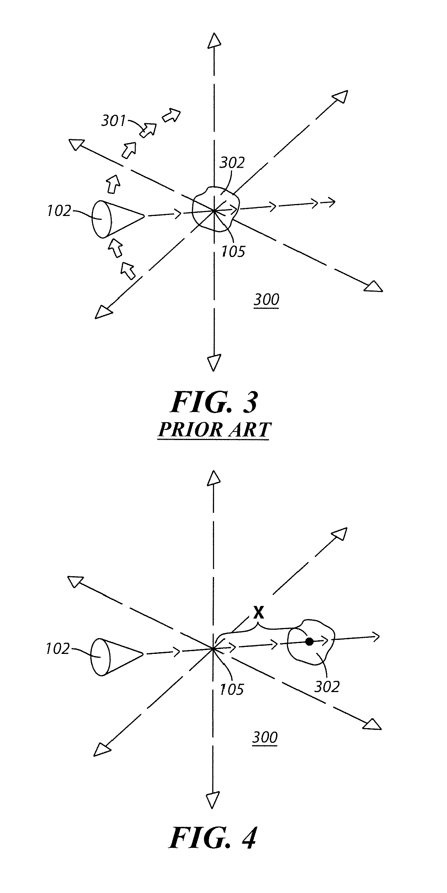

At this point it may be helpful to note some additional specific details regarding the ordinary manner of use of such a radiation-therapy machine 101. FIG. 3 presents a three-dimensional X-Y-Z coordinate system 300 that corresponds to the radiation-therapy machine 101 itself. Per typical prior art practice in these regards the radiation source 102 remains in a same plane even as the radiation source 102 assumes any number of different angles as the radiation source 102 traverses an arcuate path 301 (such as a circular path). As mentioned above, the radiation source 102 remains aimed at the isocenter 105 throughout its traversal of the arcuate path 301. Accordingly it is an ordinary practice to physically locate the patient's target volume 302 at the isocenter 105 and to maintain that position throughout the course of the radiation treatment session.

The present teachings contemplate something quite different in these regards. In particular, and referring now to FIG. 4, the control circuit 109 is configured to control the selective movement of at least the patient-support platform 108 such that during at least a part of a radiation treatment session a patient's radiation-treatment target volume 302 is maintained at a predetermined offset distance "X" away from the radiation-treatment isocenter 105. By one approach this offset distance "X" can be on the side of the isocenter 105 that is opposite the radiation source 102 (as illustrated). By another approach, if desired, this offset distance "X" can be maintained on the side of the isocenter 105 that is on the same side as the radiation source 102.

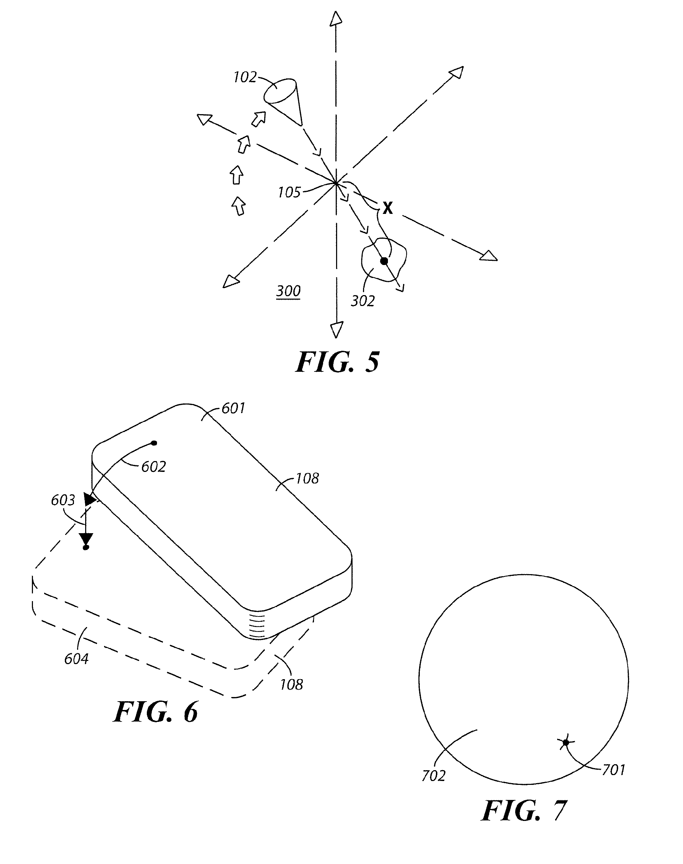

By one approach the control circuit 109 maintains the described relationships even as the radiation source 102 moves along its arcuate path 301. By way of illustration, FIG. 5 presents the radiation source 102 in an arcuately-translated position as compared to FIG. 4. Notwithstanding this movement, and notwithstanding that the radiation source 102 remains directed towards the isocenter 105 of the radiation-therapy machine 101, the patient's target volume 302 is still positioned at the same predetermined offset distance "X" away from the isocenter 105 (and is still at least substantially collinear with the radiation source's central ray).

Persisting the described relationships can be accomplished by selective movement of the patient's treatment volume with respect to the radiation-therapy machine 101. This movement can be achieved by moving the radiation-therapy machine 101 itself, or by moving the patient, or by moving both the radiation-therapy machine 101 and the patient. As the radiation head is constrained to a single plane during a treatment session the present teachings contemplate moving the patient during the treatment session to accomplish the desired movement of the patient's treatment volume.

As noted above, the control circuit 109 maintains the predetermined offset distance, at least in part, by selective movement of the patient-support platform 108. FIG. 6 offers a simple illustrative example in these regards. As illustrated, the patient-support platform 108 moves from a first position (denoted by reference numeral 601) along a first movement vector 602 and then along a second movement vector 603 to a second position (denoted by reference numeral 604. The particular movements (and the speed of those movements) employed by the control circuit 109 to achieve the desired movement of the patient's target volume 302 can vary with respect to the movement capabilities of the patient-support platform 108 itself.

Those skilled in the art will appreciate that the use of the predetermined offset distance in turn facilitates controlling the selective movement of the patient-support platform 108 such that during a radiation treatment session, and as the radiation source 102 traverses its own pathway, the patient's radiation-treatment target volume 302 moves (with respect to the isocenter 105) along a predetermined trajectory (i.e., a continguous path). By one approach this predetermined trajectory is constrained to a preselected geometric volume. If desired, this constraint can comprise constraining the predetermined trajectory to a peripheral surface of the preselected geometric volume.

Referring now to FIG. 7, and by way of a simple illustrative example, the aforementioned preselected geometric volume can comprise a sphere 702. In this example this sphere 702 has a radius equal to the aforementioned predetermined offset distance "X" and has its center coincident with the radiation-treatment isocenter 105. Reference numeral 701, in turn, represents a particular location of the patient's target volume 302 on the surface periphery of the sphere 702.

It will be understood that the entire target volume 302 will not typically fit within the space afforded by such a point on the sphere's surface. Instead, that point (and, indeed, the aforementioned predetermined offset distance "X") will typically have some predetermined assigned relationship with respect to the treatment volume. For example, by one approach point 701 on the sphere's surface can correspond to some specifically identified point as corresponds to the treatment volume (such as, but not limited to, a geometric center of the treatment volume).

As the radiation source 102 moves about the isocenter 105 during a radiation treatment session, the control circuit 109 manipulates the patient-support platform 108 to move in a way that moves the target volume 302 along a predetermined trajectory on the surface of the sphere 702. Doing this properly, of course, will, amongst other things, achieve the aforementioned maintenance of the predetermined offset distance "X" between the target volume 302 and the isocenter 105.

In addition, however, such an approach can have the salutary effect of changing the plane of the radiation source's fan beam with respect to the patient. By changing this relative plane, of course, the radiation treatment can provide a desired dose of radiation to the treatment volume while simultaneously exposing an overall greater quantity of non-targeted patient material to a same aggregated amount of untargeted radiation. As a result, any given non-targeted volume will tend to receive a smaller collateral dose as compared to prior art techniques in these regards.

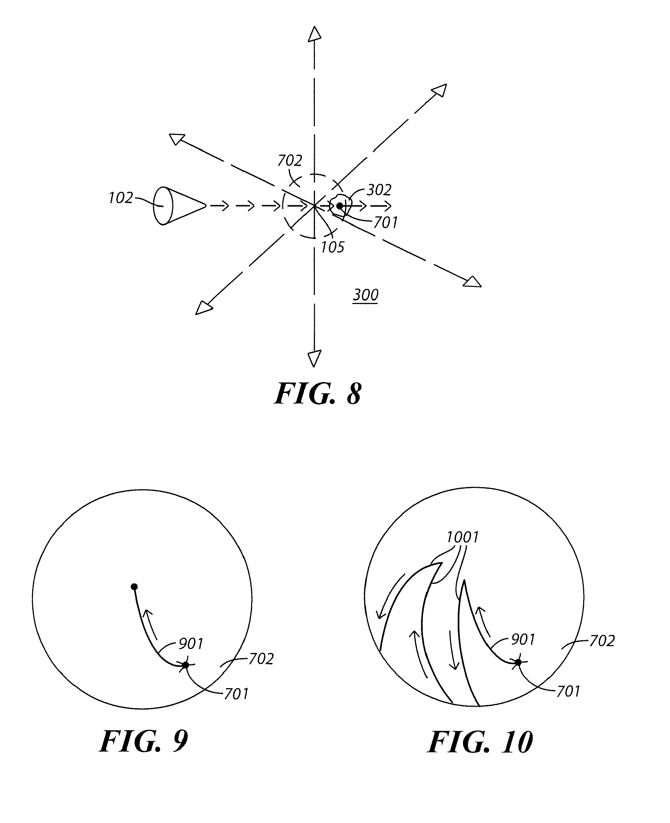

FIG. 9 illustrates a first leg 901 of such a trajectory along the surface of the sphere 702. FIG. 10 illustrates additional legs 1001 of the same trajectory. It will be noted that sometimes the trajectory can be quite linear and at other times quite nonlinear. The specific directions, lengths, and turns as comprise the trajectory are defined as a function of maintaining the aforementioned predetermined offset distance while also utilizing different exposure planes during the radiation treatment session. The specifics of a given trajectory can also be limited to ensure that the patient-support platform 108 is physically able to achieve the corresponding desired movements and also that the patient-support platform 108 has room within the radiation-therapy machine 101 to accomplish the desired movements without any collisions between the patient-support platform 108 (and/or the patient) with other objects/surfaces in the treatment zone.

As noted above, while a sphere-shaped volume serves as a useful illustrative example (and in fact offers a number of benefits in terms of simplifying the calculation of a particular trajectory for a specific radiation-treatment plan), these teachings will readily accommodate other shapes. This can include both relatively-symmetrical shapes (such as the aforementioned sphere, a cube, and so forth) as well as arbitrary and/or non-symmetrical shapes of convenience. There may be certain patients who present particular targeting challenges, for example, that are best served by seemingly-random blob-like volumes having a surface shape that happens to be particularly appropriate for a particular patient.

For a given radiation treatment session the specifics of the treatment as pertain to the shape of the aforementioned volume, the predetermined offset distance, the trajectory to employ, and other corresponding system parameters and settings are specified by the corresponding radiation treatment plan. As is known in the art. treatment plans typically serve to specify any number of operating parameters as pertain to the administration of such a treatment with respect to a given patient. For example, many treatment plans provide for exposing the target volume to possibly-varying dosages of radiation from a number of different directions using variable beam shapes.

Such treatment plans are often optimized prior to use. (As used herein, "optimization" will be understood to refer to improving upon a candidate treatment plan without necessarily ensuring that the optimized result is, in fact, the singular best solution.) Many optimization approaches use an automated incremental methodology where various optimization results are calculated and tested in turn using a variety of automatically-modified (i.e., "incremented") treatment plan optimization parameters. Such an approach can serve well in the present regards.

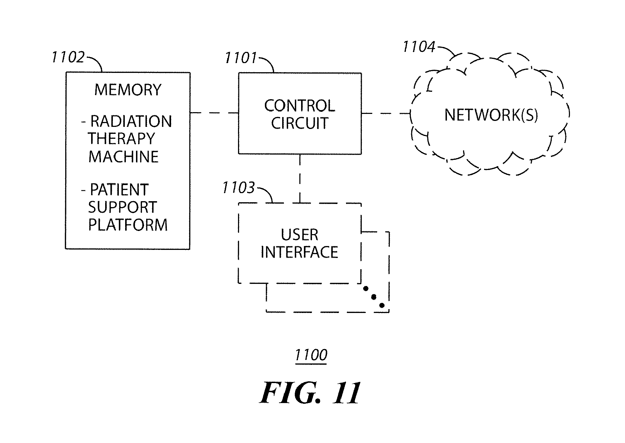

Referring to FIG. 11, a radiation-therapy treatment-planning apparatus 1100 can comprise a corresponding control circuit 1101 (such as a suitably programmed and provisioned computer(s)) that operably couples to a memory 1102. This memory 1102 can have information stored therein such as information regarding one or more available radiation-therapy machines having a movable radiation source and a corresponding radiation-treatment isocenter as well as a patient-support platform (such as those described herein). Via programming that employs standard optimization techniques the control circuit 1101 can test a variety of volume shapes, predetermined offset distances, trajectories, and so forth to identify one or more suitable treatment plans that achieve the specified dosing of a particular patient's treatment volume while avoiding to some considerable or specified extent undue dosing of untargeted volumes.

So configured, the control circuit 1101 can optimize a radiation-therapy treatment plan that specifies a treatment session that includes controlling selective movement of a patient-support platform such that during a least a portion of the planned radiation treatment session the patient's radiation-treatment target volume is maintained at a predetermined offset distance away from the radiation-treatment isocenter. Such a plan can further specify, as described above, a particular trajectory corresponding to relative movement of the treatment volume with respect to the radiation-treatment machine's isocenter, which trajectory can be constrained as appropriate to the surface of a geometric volume of choice per the foregoing.

If and as desired, such a control circuit 1101 can optionally operably couple to one or more user interfaces 1103 of choice. This user interface 1103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

Such a control circuit 1101 can also optionally communicatively couple to one or more networks 1104 including any of a variety of intranets and extranets (such as but not limited to the Internet). So configured, the control circuit 1101 can, for example, share a particular optimized radiation-treatment plan with other users, facilities, or the like.

So configured, a patient (and hence the patient's target volume) moves via a predetermined series of patient-support platform movements with respect to a radiation-treatment machine to thereby greatly facilitate exposing the target volume to radiation fan beams in more than a single plane during the course of a single treatment session. These teachings will accommodate a wide variety of modifications to thereby meet any number of challenges and requirements that are, at present, difficult or impossible to accomplish.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept. As but one example in these regards, if desired a single radiation-treatment session could use, during a first portion of the session, a first set of selected parameters for the aforementioned predetermined offset distance, predetermined trajectory, and shape of the preselected geometric volume, and then switch to using, during a second subsequent portion of the session, a second set of different selected parameters for these particular settings and selections.

## Claims

1. A radiation-therapy apparatus comprising:
a radiation-therapy machine having a movable radiation source and a corresponding radiation-treatment isocenter;
a patient-support platform;
a control circuit configured to control selective movement of at least the patient-support platform such that during at least a part of a radiation treatment session a patient's radiation-treatment target volume is maintained at a predetermined offset distance away from the radiation-treatment isocenter.

2. The radiation-therapy apparatus of claim 1 wherein the control circuit is configured to maintain the predetermined offset distance throughout the radiation treatment session for the patient.

3. The radiation-therapy apparatus of claim 1 wherein the control circuit is further configured to control selective movement of at least the patient-support platform such that during the radiation treatment session the patient's radiation-treatment target volume moves along a predetermined trajectory.

4. The radiation-therapy apparatus of claim 3 wherein the predetermined trajectory is constrained to a peripheral surface of a preselected geometric volume.

5. The radiation-therapy apparatus of claim 4 wherein the preselected geometric volume comprises a sphere.

6. The radiation-therapy apparatus of claim 5 wherein the sphere has its center coincident with the radiation-treatment isocenter and its radius is equal to the predetermined offset distance.

7. A radiation-therapy treatment-planning apparatus comprising:
a memory having information stored therein regarding a radiation-therapy machine having a movable radiation source and a corresponding radiation-treatment isocenter and a patient-support platform;
a control circuit operably coupled to the memory and configured to optimize a radiation-therapy treatment session by, at least in part, controlling selective movement of the patient-support platform such that during at least a part of a planned radiation treatment session a patient's radiation-treatment target volume is maintained at a predetermined offset distance away from the radiation-treatment isocenter.

8. The radiation-therapy treatment-planning apparatus of claim 7 wherein the control circuit is further configured to optimize the radiation-therapy treatment session by, at least in part, maintaining the predetermined offset distance throughout the planned radiation treatment session for the patient.

9. The radiation-therapy treatment-planning apparatus of claim 7 wherein the control circuit is further configured to optimize the radiation-therapy treatment session by, at least in part, controlling selective movement of the patient-support platform such that during the planned radiation treatment session the patient's radiation-treatment target volume moves along a predetermined trajectory.

10. The radiation-therapy treatment-planning apparatus of claim 9 wherein the predetermined trajectory is constrained to a preselected geometric volume.

11. The radiation-therapy treatment-planning apparatus of claim 10 wherein the predetermined trajectory is constrained to a peripheral surface of the preselected geometric volume.

12. The radiation-therapy treatment-planning apparatus of claim 11 wherein the preselected geometric volume comprises a sphere.

13. The radiation-therapy treatment-planning apparatus of claim 12 wherein the sphere has its center coincident with the radiation-treatment isocenter and its radius is equal to the predetermined offset distance.

14. Any of claims 1 through 13 wherein the predetermined offset distance is at least ten centimeters.

15. A method comprising using the control circuit of any of claims 1 to 6 to control selective movement of a patient-support platform such that during at least a part of the radiation treatment session a patient's radiation-treatment target volume is maintained at a predetermined offset distance away from the radiation-treatment isocenter.
